(19) Europäisches Patentamt
European Patent Office
Office européen des brevets

(11) **EP 4 595 991 A1**

(12) **EUROPEAN PATENT APPLICATION**

(43) Date of publication:
**06.08.2025 Bulletin 2025/32**

(21) Application number: **25166428.0**

(22) Date of filing: **14.09.2018**

(51) International Patent Classification (IPC):
**A61M 1/00** (2006.01)

(52) Cooperative Patent Classification (CPC):
**A61M 1/76; A61M 1/84; A61M 1/86;** A61M 2206/20

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **14.03.2018 US 201862642693 P**

(62) Document number(s) of the earlier application(s) in
accordance with Art. 76 EPC:
**18792579.7 / 3 765 107**

(71) Applicant: **Conmed Corporation
Largo, FL 33773 (US)**

(72) Inventor: **SNYDER, Jessica
Poland, NY 13431 (US)**

(74) Representative: **Strehl & Partner mbB
Maximilianstrasse 54
80538 München (DE)**

Remarks:
This application was filed on 26.03.2025 as a
divisional application to the application mentioned
under INID code 62.

(54) **SURGICAL SUCTION SYSTEM**

(57) A surgical suction system. The surgical suction system includes a large bore Yankauer suction device having a distal tip with a first diameter, and a proximal end with exterior hose barbs and a second diameter. The second diameter is greater than the first diameter such that a channel extending through the large bore Yankauer suction device may have an increasing diameter from the distal tip to the proximal end. The surgical system also includes cannulated tubing having a distal end and a proximal end, the distal end configured to mate with the exterior hose barbs of the Yankauer suction device. The surgical system further includes an adapter having a distal end with exterior hose barbs and a proximal end. The exterior hose barbs configured to mate with the proximal end of the cannulated tubing. The proximal end of the adapter is configured to connect to a port to a canister, wall vacuum, or other biological material collection device.

FIG. 3

**EP 4 595 991 A1**

## Description

CROSS-REFERENCE TO RELATED APPLICATIONS

This application claims priority to U. S. Provisional Patent Application Serial No. 62/642693, filed on March 14, 2018 and entitled "Large Bore Yankauer Kit."

BACKGROUND OF THE INVENTION

### 1. Field of the Invention

The present invention is directed generally to medical devices used during surgical procedures and, more particularly, to a surgical suction system with adapters for interchanging instruments and connecting to a variety of vacuum systems and collection devices.

### 2. Description of Related Art

During the performance of various medical procedures it frequently becomes necessary to remove material such as blood and bone chips during an orthopedic procedure, by suctioning. Conventional suction canisters connected to the suction instruments used in medical procedures have standard fittings for receiving suction tubing. Conventional suction tubing has an inner diameter typically within the range of $\frac{1}{8}$ to $\frac{9}{32}$ inch. However, in many orthopedic procedures, the nature of the materials being removed by the suctioning are such that the materials clog the standard tubing, instruments, and fittings used for connecting suction tubing.

In a single standard hip or knee replacement (or revision) procedure, a surgeon may unclog the suction tubing up to 15 times. As mentioned above, the suction tubing can become clogged with a mixture of solid and liquid biological materials, such as blood, fat, bone chips, and coagulated blood. To unclog the suction system, the surgeon has to disassemble and reassemble the components of the system. Thus, unclogging the suction system is labor intensive and time consuming.

In some cases, the surgical procedure may require use of a small suction instrument. For example, a small suction instrument may be required for small incisions made at the beginning of a surgical procedure when the surgical site is small and the suction is used for liquid biological materials only. Currently, small and large suction instruments are components of their own separate suction systems. In other words, a surgeon must switch suction systems to use a different sized suction instrument.

Accordingly, there is a need for a suction system that minimizes clogging and allows the interchangeable use of small and large suction instruments.

SUMMARY OF THE INVENTION

The present invention is directed to a surgical suction system with adapters for interchanging instruments and connecting to a variety of vacuum systems and collection devices. According to one aspect, the suction system includes a Yankauer suction device having a distal tip with a first diameter, and a proximal end with exterior hose barbs (or, alternatively, threads) and a second diameter. The second diameter is greater than the first diameter. The suction system also includes cannulated tubing having a distal end and a proximal end. The distal end of the cannulated tubing is configured to mate with the exterior hose barbs of the Yankauer suction device. The suction system further includes an adapter having a distal end with exterior hose barbs and a proximal end. The exterior hose barbs of the adapter are configured to mate with the proximal end of the cannulated tubing.

According to another aspect, the suction system includes a Yankauer suction device having a distal tip with a first diameter, and a proximal end with exterior hose barbs and a second diameter. The second diameter is greater than the first diameter. The suction system also includes cannulated tubing having a distal end and a proximal end. The distal end of the cannulated tubing is configured to mate with the exterior hose barbs of the Yankauer suction device. The suction system further includes a first adapter having a distal end with exterior hose barbs and a proximal end. The exterior hose barbs of the adapter are configured to mate with the proximal end of the cannulated tubing. The suction system also includes a second instrument having a proximal end, which is configured to mate with a proximal end of a second adapter. The second adapter has a proximal end with exterior hose barbs, which are configured to mate with the distal end of the cannulated tubing. The Yankauer suction device and the second instrument are interchangeable. In a first configuration, the proximal end of the Yankauer suction device is connected to the distal end of the cannulated tubing. In a second configuration, the proximal end of the second instrument is connected to the distal end of the second adapter and the proximal end of the second adapter is connected to the distal end of the cannulated tubing.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein. It should also be appreciated that terminology explicitly employed herein that also may appear in any disclosure incorporated by reference should be accorded a meaning most consistent with the particular concepts disclosed herein.

These and other aspects of the invention will be apparent from and elucidated with reference to the embodiment(s) described hereinafter.

BRIEF DESCRIPTION OF THE DRAWINGS

One or more aspects of the present invention are particularly pointed out and distinctly claimed as examples in the claims at the conclusion of the specification. The foregoing and other objects, features, and advantages of the invention are apparent from the following description taken in conjunction with the accompanying drawings in which:

FIG. 1 is an exploded side view schematic representation of the suction system, according to an embodiment;
FIG. 2 is a front (distal) view schematic representation of the large bore Yankauer suction device, according to an embodiment.
FIG. 3 is a side interior view schematic representation of the suction system, according to an embodiment.
FIG. 4 is a perspective side view schematic representation of the flexible adapter, according to an embodiment.
FIG. 5 is a back (proximal) perspective view schematic representation of the flexible adapter, according to embodiment.
FIG. 6 is a side view schematic representation of the flexible adapter, according to an embodiment;
FIG. 7 is a cross-sectional view schematic representation of the flexible adapter of FIG. 6; and
FIG. 8 is a perspective view schematic representation of a pour spout adapter, according to an embodiment.

DETAILED DESCRIPTION OF THE INVENTION

Aspects of the present invention and certain features, advantages, and details thereof, are explained more fully below with reference to the non-limiting examples illustrated in the accompanying drawings. Descriptions of well-known structures are omitted so as not to unnecessarily obscure the invention in detail. It should be understood, however, that the detailed description and the specific non-limiting examples, while indicating aspects of the invention, are given by way of illustration only, and are not by way of limitation. Various substitutions, modifications, additions, and/or arrangements, within the spirit and/or scope of the underlying inventive concepts will be apparent to those skilled in the art from this disclosure.

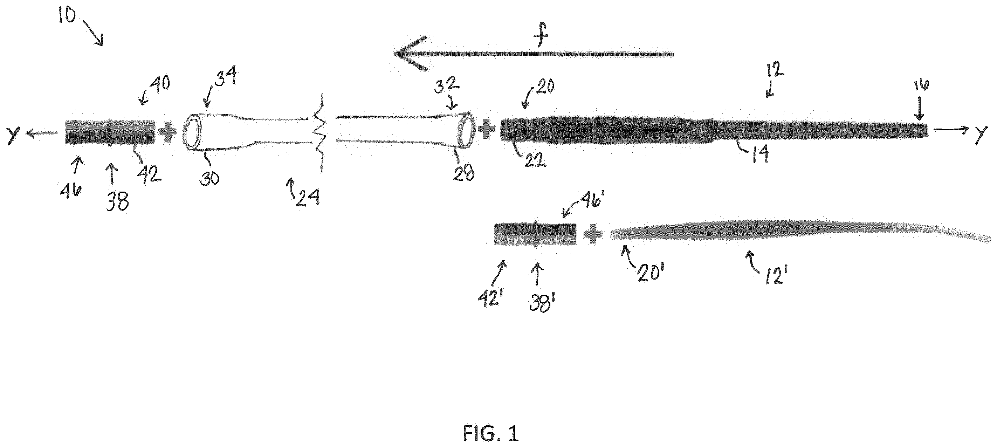

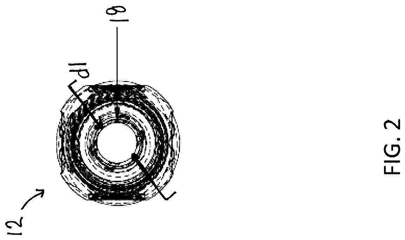

Referring now to the figures, wherein like reference numerals refer to like parts throughout, FIG. 1 shows an exploded side view schematic representation of the suction system 10, according to an embodiment. The suction system 10 comprises a large bore suction instrument 12, such as a Yankauer suction tool/device (hereinafter "large bore Yankauer"). The large bore Yankauer 12 comprises a body 14 with a distal tip 16 having a bore 18 extending therethrough, as shown in FIG. 2. In the depicted embodiment, the bore 18 is relatively large in order to remove both liquid and solid biological materials (e.g., blood, fat, bone chips) from the surgical site. In one embodiment, the bore 18 has an inner diameter $d1$ of 6.00 mm; however, a bore 18 of an alternative size may be used as long as the inner diameter is sufficiently large to allow for the passage of both liquid and solid biological materials. Specifically, as used herein "large bore" is defined to include a broad range of bore sizes having a diameter within the range of 5-10 mm, with the inner diameter $d1$ of the bore 18 preferably within the range of 6-8 mm.

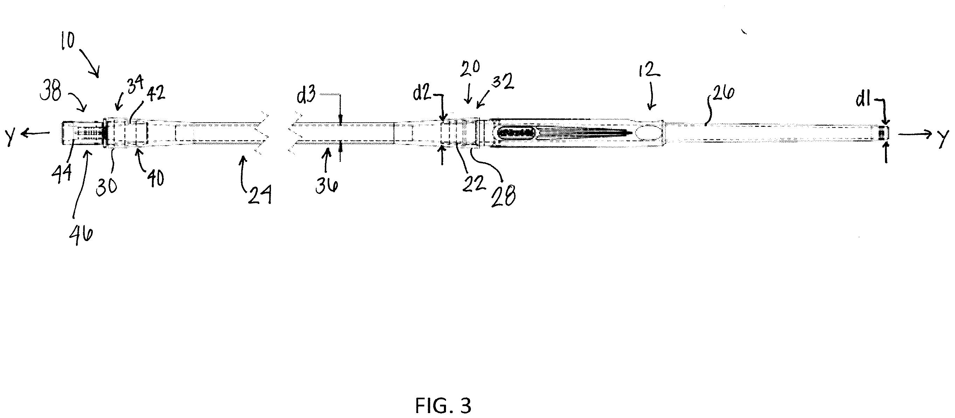

Referring back to FIG. 1, the body 14 of the large bore Yankauer 12 extends from its distal tip 16 to a proximal end 20 along a central longitudinal y - y axis. The proximal end 20 of the large bore Yankauer 12 comprises exterior hose barbs 22. Hose barbs 22 are understood by one of ordinary skill in the art as barb-like rings which allow for each push-connection and prevent easy disconnection to tubing. The exterior hose barbs 22 at the proximal end 20 are sized and configured to removably attach to cannulated flexible tubing 24, as shown in FIG. 3. As also shown in FIG. 3, a channel 26 extends along the central longitudinal y - y axis through the body 14 of the large bore Yankauer 12 from the bore 18 at the distal tip 16 to the proximal end 20. In one embodiment, the inner diameter of the channel 26 is smallest at the distal tip 16 (i.e., $d1$ of bore 18 in FIG. 2). For example, in one embodiment, the inner diameter of the channel 26 increases from the inner diameter $d1$ of the distal tip 16 to the inner diameter $d2$ (FIG. 3) at the proximal end 20. Increasing the inner diameter of the large bore Yankauer 12 from distal tip 16 to the proximal end 20 with the direction of suction $f$ (FIG. 1) provides extra space for flowing biological materials and minimizes clogs.

As stated above, the proximal end 16 of the large bore Yankauer 12 is sized and configured to mate with or otherwise connect to the cannulated flexible tubing 24. In the embodiment shown in FIG. 1, the cannulated flexible tubing 24 comprises a pair of flared ends 28, 30, one at a distal end 32 of the flexible tubing 24 and one at a proximal end 34 of the flexible tubing 24, respectively. The flared ends 28, 30 are portions of the flexible tubing 24 wherein the flexible tubing 24 is tapered or otherwise has a changing inner diameter. The flared ends 28, 30 accommodate connections between the flexible tubing 24 and instruments and/or connectors, and are configured and/or structured to prevent pinching or kinking of the tube adjacent to the flared end (conventional tubing without the flared ends kink or pinch at a section adjacent the connected ends of the tube). Accordingly, other dimensions and configurations of the flexible tubing 24 may be used as long as the distal and proximal ends 32, 34 are suitable for stable connection to the instruments and/or connectors.

In the embodiment depicted in FIG. 1, an inner diameter of the cannulated flexible tubing 24 increases toward the distal end 32, creating the first flared end 28 and increases in the opposite direction toward the proximal end 34, creating the second flared end 30. As the inner diameter of the cannulated flexible tubing 24 increases toward both the distal and proximal ends 32, 34, the inner diameter of the flexible tubing 24 is smallest at a central portion 36 of the flexible tubing 24 between the distal and proximal ends 32, 34, as shown in FIG. 3. In FIG. 3, the central portion 36 extends to distal and proximal ends 32, 34 and has an inner diameter $d3$, which is greater than the inner diameter $d1$ of the bore 18 at the distal tip 16 of the large bore Yankauer 12. For example, in one embodiment, the inner diameter $d1$ of the first bore is 6.00 mm and the inner diameter $d3$ of the central portion 36 of the flexible tubing 24 is 8.38 mm. In an alternative embodiment, $d3 > d2 > d1$ such that the inner diameter of the suction system 10 increase with the direction of suction $f$ (FIG. 1). In another embodiment, the inner diameter $d3$ of the central portion 36 is 11/32 inch, which is larger than the inner diameter of tubing generally used with Yankauer suction devices. A relatively large inner diameter of the flexible tubing 24 increases flow in the direction of suction $f$ (FIG. 1), which minimizes clogging.

Still referring to FIG. 3, the first flared end 28 of the cannulated flexible tubing 24 is sized and configured to removably receive the proximal end 20 of the large bore Yankauer 12. In particular, FIG. 3 shows that the first flared end 28 engages or catches on each of the barbs 22 on the proximal end 20 of the large bore Yankauer 12. The second flared end 30 of the cannulated flexible tubing 24 is sized and configured to removably receive a flexible adapter 38 (also in FIG. 1). As shown in FIG. 1, the flexible adapter 38 comprises a distal end 40 with exterior hose barbs 42. The exterior hose barbs 42 are sized and configured to mate with or otherwise connect to the second flared end 30 of the cannulated flexible tubing 24, as shown in FIG. 3. In the depicted embodiment, the second flared end 30 engages or catches on each of the barbs 42 on the distal end 40 of the flexible adapter 38.

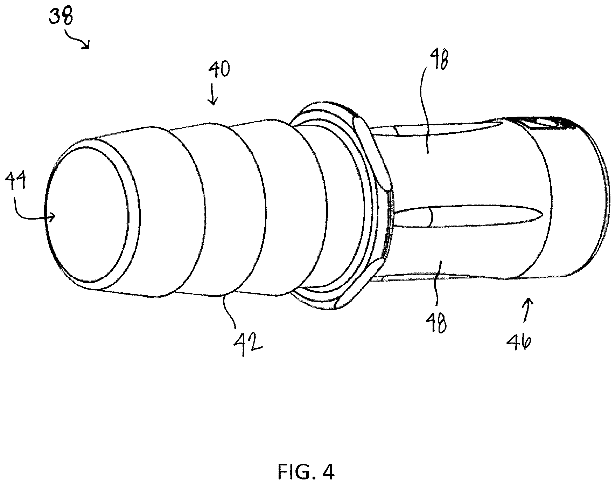

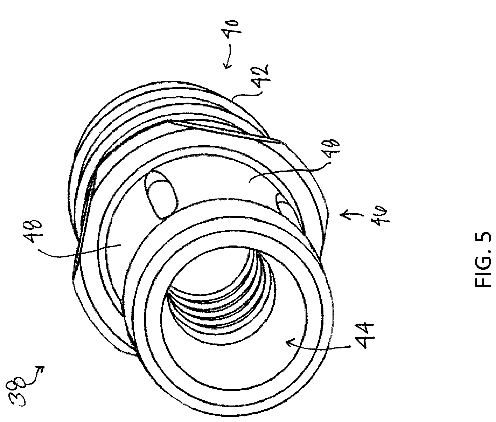

Referring now to FIGs. 4-5, there are shown various views schematic representations of the flexible adapter 38, according to an embodiment. FIG. 4 shows a perspective side view of the flexible adapter 38. In the depicted embodiment, the flexible adapter 38 has exterior hose barbs 42 on its distal end 40, as described above. In one embodiment, the exterior hose barbs 42 of the flexible adapter 38 match or are substantially similar to the exterior hose barbs 22 at the proximal end 20 of the large bore Yankauer 12. FIG. 5 shows a perspective back (proximal) view schematic representation of the flexible adapter 38. FIGs. 4 and 5 show a bore 44 extending from a proximal end 46 to the distal end 40 of the flexible adapter 38. FIG. 3 also shows the bore 44 extending therethrough. When assembled, the suction system 10 comprises a pathway for biological materials from the distal tip 16 of the large bore Yankauer 12 to a proximal end 46 of the flexible adapter 38. The pathway includes the bore 18 and channel 26 in the large bore Yankauer 12, the cannulated flexible tubing 24, and the bore 44 of the flexible adapter 38, as shown in FIG. 3.

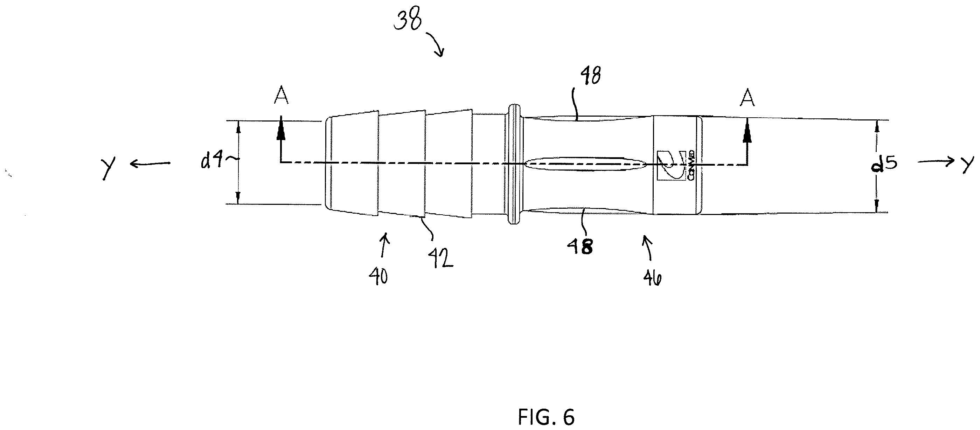

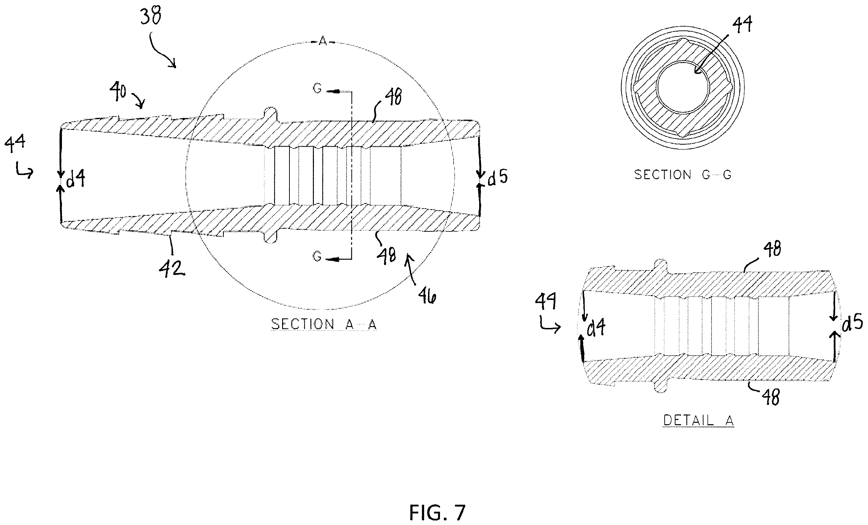

FIGs. 6-7 also show various views schematic representations of the flexible adapter 38, according to an embodiment. FIG. 6 shows a side view schematic representation of the flexible adapter 38, while FIG. 7 shows a cross-sectional view schematic representation of the flexible adapter 38 of FIG. 6. In the depicted embodiment, as described above, the bore 44 extends from the proximal end 46 to the distal end 40 of the flexible adapter 38. In one embodiment, the smallest inner diameter of the flexible adapter 38 is larger than the inner diameter $d1$ of the bore 18 of the distal tip 16 of the large bore Yankauer 12. For example, the smallest inner diameter of the flexible adapter 38 is 7.24 mm, while the inner diameter $d1$ of the bore 18 of the distal tip 16 of the large bore Yankauer 12 is 6.00 mm.

In an embodiment, the inner diameter $d4$ at the distal end 40 of the flexible adapter 38 is smaller than the inner diameter $d5$ at the proximal end 46 of the flexible adapter 38. In some embodiments, the largest inner diameter of the suction system 10 is the inner diameter $d5$ at the proximal end 46 of the flexible adapter 38 such that the inner diameter of the suction system 10 increases from the distal tip 16 of the large bore Yankauer 12 to the proximal end 46 of the flexible adapter 38. An increasing inner diameter of the suction system 10 in the direction of suction $f$ (FIG. 1) allows for biological materials of varying sizes and consistencies to pass through the suction system 10, increasing flow to the proximal end 46 and minimizing clogs. In an alternative embodiment shown in FIGs. 6-7, the inner diameter $d4$ at the distal end 40 of the flexible adapter 38 is larger than the inner diameter $d5$ at the proximal end 46 of the flexible adapter 38. For example, the inner diameter $d4$ at the distal end 40 is 11.43 mm, while the inner diameter $d5$ at the proximal end 46 is 9.73 mm.

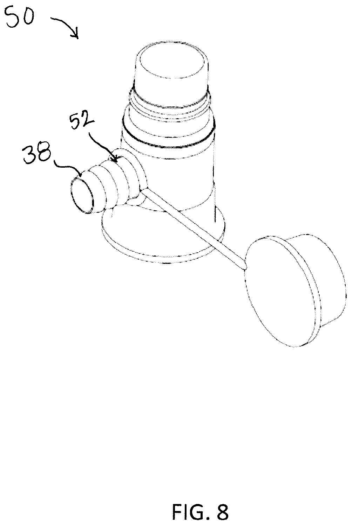

Referring back to FIG. 4, the proximal end 46 of the flexible adapter 38 is configured to interface the cannulated flexible tubing 24 with a suction collection canister, other container, or existing instruments having a relatively small diameter (as described in detail below, and as should be understood by a person of ordinary skill in the art in conjunction with a review of this disclosure). For example, the proximal end 46 of the flexible adapter 38 can be connected to a port on a collection canister, such as a collection canister on a cart (e.g., portable vacuum system). In another example, the cannulated flexible tubing 24 is configured to interface with a flexible adapter 38 which is fixed (permanently or removably) to a port 52 on a pour spout adapter 50 for a collection canister, as shown in FIG. 8. As understood by one of ordinary skill in the art, the pour spout adapter 50 connects to a suction collection canister (not shown) so that biological material removed by the suction system 10 can be pour from the collection canister without removal of its lid (or other covering). In another embodiment, the proximal end 46 of the flexible adapter 38 is configured to connect to a port for a wall vacuum. The proximal end 46 of the flexible adapter 38 can be connected to a port in any of the examples described above as understood by one of ordinary skill in the art.

As shown in FIG. 4-7, the proximal end 46 of the flexible adapter 38 has one or more flats 48. In the depicted embodiment, the flats 48 are spaced and extend along the length of the flexible adapter 38. The flats 48 are configured and/or structured to prevent the flexible adapter 38 from rolling off the instrument table or other surface when the flexible adapter 38 (or suction system 10) is not in use. In an embodiment, the flats 48 extend in a direction substantially parallel to the central longitudinal axis y - y extending through the flexible adapter 38.

Turning back to FIG. 1, an additional flexible adapter 38' can be used to connect a small bore Yankauer 12' with the first flared end 32 (i.e., distal end 28) of the cannulated flexible tubing 24. As mentioned above, the flexible adapter 38' can be used to connect the flexible tubing 24 to a smaller instrument (relative to the large bore Yankauer 12). It is important to note that when the additional flexible adapter 38' is used, it is the same as the flexible adapter shown in FIGs. 4-7; however, the distal end 40 of the flexible adapter 38 is the same as the proximal end 40' of the additional flexible adapter 38' and the proximal end 46 of the flexible adapter 38 is the same as the distal end 46' of the additional flexible adapter 38'. In other words, in one embodiment, the additional flexible adapter 38' is a rotated configuration (180°) of the flexible adapter 38 in FIGs. 4-7.

As shown in FIG. 1, the distal end 46' of the flexible adapter 38' connects to a proximal end 20' of the small bore Yankauer 12' (or other small instrument) and the proximal end 42' of the flexible adapter 38' connects to the first flared end 32 of the flexible tubing 24. Thus, the flexible adapter 38' and connected small bore Yankauer 12' can be interchanged with the large bore Yankauer 12, as shown in FIG. 1. The small bore Yankauer 12' can be used to suction or remove liquids from surgical areas, such as small incisions. The ability to quickly interchange the large bore Yankauer 12 with a smaller instrument 12' allows the user to select the instrument (e.g., large bore Yankauer 12 or small bore Yankauer 12') based on factors, such as the size of the surgical site and the biological materials that are being removed from the surgical site.

All definitions, as defined and used herein, should be understood to control over dictionary definitions, definitions in documents incorporated by reference, and/or ordinary meanings of the defined terms.

While various embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the teachings is/are used. Those skilled in the art will recognize, or be able to ascertain using no more than routine experimentation, many equivalents to the specific embodiments described herein. It is, therefore, to be understood that the foregoing embodiments are presented by way of example only and that, within the scope of the appended claims and equivalents thereto, embodiments may be practiced otherwise than as specifically described and claimed. Embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the scope of the present disclosure.

The terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the terms "comprise" (and any form of comprise, such as "comprises" and "comprising"), "have" (and any form of have, such as, "has" and "having"), "include" (and any form of include, such as "includes" and "including"), and "contain" (any form of contain, such as "contains" and "containing") are open-ended linking verbs. As a result, a method or device that "comprises", "has", "includes" or "contains" one or more steps or elements. Likewise, a step of method or an element of a device that "comprises", "has", "includes" or "contains" one or more features possesses those one or more features, but is not limited to possessing only those one or more features. Furthermore, a device or structure that is configured in a certain way is configured in at least that way, but may also be configured in ways that are not listed.

The corresponding structures, materials, acts and equivalents of all means or step plus function elements in the claims below, if any, are intended to include any structure, material or act for performing the function in combination with other claimed elements as specifically claimed. The description of the present invention has been presented for purposes of illustration and description, but is not intended to be exhaustive or limited to the invention in the form disclosed. Many modifications and variations will be apparent to those of ordinary skill in the art without departing from the scope and spirit of the invention. The embodiment was chosen and described in order to best explain the principles of one or more aspects of the invention and the practical application, and to enable others of ordinary skill in the art to understand one or more aspects of the present invention for various embodiments with various modifications as are suited to the particular use contemplated.

IMPORTANT ASPECTS OF THE PRESENT INVENTION

[1] A suction system, comprising:

a Yankauer suction device having a distal tip with a first diameter and a proximal end, the proximal end having a second diameter;
wherein the second diameter is greater than the first diameter;
cannulated tubing having a distal end and a proximal end, the distal end configured to mate with the proximal end of the Yankauer suction device;
an adapter having a distal end and a proximal end, the distal end configured to mate with the proximal end of the cannulated tubing.

**[2]** The suction system of [1], further comprising a channel extending from the distal tip of the Yankauer suction device to the proximal end of the Yankauer suction device, the channel having an inner diameter increasing from the distal tip to the proximal end.

**[3]** The suction system of [1], further comprising a channel extending from the distal tip of the Yankauer suction device to the proximal end of the adapter, the channel having an inner diameter increasing from the distal tip of the Yankauer suction device to the proximal end of the adapter.

[4] The suction system of [1], wherein the cannulated tubing has a third diameter greater than the first diameter.

[5] The suction system of [1], wherein the distal end of the cannulated tubing is a first flared end and the proximal end of the cannulated tubing is a second flared end.

[6] The suction system of [5], wherein an inner diameter of the cannulated tubing increases from a central position toward the first flared end and toward the second flared end.

[7] The suction system of [1], wherein the distal end of the adapter has a third diameter, which is greater than the first diameter of the distal tip of the Yankauer suction device.

[8] The suction system of [7], wherein the proximal end of the adapter has a fourth diameter, which is greater than the first diameter of the distal tip of the Yankauer suction device.

[9] The suction system of [8], wherein the fourth diameter is greater than the third diameter.

[10] The suction system of [1], further comprising one or more flats extending along the length of the proximal end of the adapter.

[11] The suction system of [1], wherein the distal end of the adapter includes exterior hose barbs and the proximal end of the Yankauer suction device have exterior hose barbs, and wherein the exterior hose barbs of the adapter match the exterior hose barbs on the proximal end of the Yankauer suction device.

**[12]** A suction system, comprising:

a Yankauer suction device having a distal tip with a first diameter and a proximal end, the proximal end having a second diameter;
wherein the second diameter is greater than the first diameter;
cannulated tubing having a distal end and a proximal end, the distal end configured to mate with the proximal end of the Yankauer suction device;
a first adapter having a distal end and a proximal end, the distal end configured to mate with the proximal end of the cannulated tubing;
a second instrument having a proximal end configured to mate with a distal end of a second adapter, the second adapter having a proximal end configured to mate with the distal end of the cannulated tubing; and
wherein the Yankauer suction device and the second instrument are interchangeable such that in a first configuration, the proximal end of the Yankauer suction device is connected to the distal end of the cannulated tubing, and in a second configuration, the proximal end of the second instrument is connected to the distal end of the second adapter, which is connected to the distal end of the cannulated tubing.

[13] The suction system of [12], wherein the second instrument is a small bore Yankauer suction device.

[14] The suction system of [12], wherein the second instrument has a smaller inner diameter than an inner diameter of the Yankauer suction device.

[15] The suction system of [12], further comprising a channel extending from the distal tip of the Yankauer suction device to the proximal end of the Yankauer suction device, the channel having an inner diameter increasing from the distal tip of the Yankauer suction device to the proximal end of the Yankauer suction device.

[16] The suction system of [12], further comprising, in the first configuration, a channel extending from the distal tip of the Yankauer suction device to the proximal end of the adapter, the channel having an inner diameter increasing from the distal tip of the Yankauer suction device to the proximal end of the adapter.

[17] The suction system of [12], wherein in the first configuration, the direction of suction is from the distal tip of the Yankauer suction device to the proximal end of the first adapter.

[18] The suction system of [12], wherein in the second configuration, the direction of suction is from the second instrument to the proximal end of the first adapter.

[19] The suction system of [12], wherein an inner diameter of the cannulated tubing increases from a central position toward its distal end and toward its proximal end.

[20] The suction system of [12], wherein the distal end of the first adapter, the distal end of the second adapter, and the proximal end of the Yankauer suction device each includes exterior hose barbs, and wherein the exterior hose barbs of the first adapter match the exterior hose barbs on the second adapter and the exterior hose barbs on the proximal end of the Yankauer suction device.

**Claims**

1.  A suction system (10), comprising:

    a Yankauer suction device (12) having a distal tip (16) with a first diameter (d1) and a proximal end (20), the proximal end (20) having a second diameter (d2);
    wherein the second diameter (d2) is greater than the first diameter (d1);
    cannulated tubing (24) having a distal end (32) and a proximal end (34), the distal end (32) configured to mate with the proximal end (20) of the Yankauer suction device (12);
    a first adapter (38) having a distal end (40) and a proximal end (46), the distal end (32) configured to mate with the proximal end (34) of the cannulated tubing (24);
    a second instrument (12') having a proximal end (20') configured to mate with a distal end (46') of a second adapter (38'), the second adapter (38') having a proximal end (42') configured to mate with the distal end (32) of the cannulated tubing (24); and
    wherein the Yankauer suction device (12) and the second instrument (12') are interchangeable such that in a first configuration, the proximal end (20) of the Yankauer suction device (12) is connected to the distal end (32) of the cannulated tubing (24), and in a second configuration, the proximal end (42') of the second instrument (12') is connected to the distal end (46') of the second adapter (38'), which is connected to the distal end (32) of the cannulated tubing (24).

2.  The suction system (10) of claim 1, wherein the second instrument (12') is a small bore Yankauer suction device.

3.  The suction system (10) of claim 1 or 2, wherein the second instrument (12') has a smaller inner diameter than an inner diameter of the Yankauer suction device (12).

4.  The suction system (10) of any preceding claim, further comprising a channel (26) extending from the distal tip (16) of the Yankauer suction device (12) to the proximal end (20) of the Yankauer suction device (12), the channel (26) having an inner diameter increasing from the distal tip (16) of the Yankauer suction device (12) to the proximal end (20) of the Yankauer suction device (12).

5.  The suction system (10) of any of claims 1 to 3, further comprising, in the first configuration, a channel (26) extending from the distal tip (16) of the Yankauer suction device (12) to the proximal end (46) of the adapter (38), the channel (26) having an inner diameter increasing from the distal tip (16) of the Yankauer suction device (12) to the proximal end (46) of the adapter (38).

6.  The suction system (10) of any preceding claim, wherein in the first configuration, the direction of suction is from the distal tip (16) of the Yankauer suction device (12) to the proximal end (46) of the first adapter (38).

7.  The suction system (10) of any preceding claim, wherein in the second configuration, the direction of suction is from the second instrument (12') to the proximal end (46) of the first adapter (38).

8.  The suction system (10) of any preceding claim, wherein an inner diameter of the cannulated tubing (24) increases from a central position (36) toward its distal end (32) and toward its proximal end (34).

9.  The suction system (10) of any preceding claim, wherein

    the distal end (32) of the first adapter (38), the distal end (46') of the second adapter (38'), and the proximal end (20) of the Yankauer suction device (12) each includes exterior hose barbs (22, 42), and
    the exterior hose barbs (42) of the first adapter (38) match the exterior hose barbs on the second adapter (38') and the exterior hose barbs (22) on the proximal end (20) of the Yankauer suction device (12).

FIG. 1

EP 4 595 991 A1

FIG. 2

FIG. 3

FIG. 4

FIG. 5

EP 4 595 991 A1

FIG.6

38

40

44

d4

42

G

G

A

48

48

46

SECTION A—A

44

d5

44

SECTION G—G

48

44

d4

48

d5

DETAIL A

FIG. 7

EP 4 595 991 A1

FIG. 8

**EUROPEAN SEARCH REPORT**

Application Number

EP 25 16 6428

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 5 921 970 A (VANDENBERG JAMES T [US]) 13 July 1999 (1999-07-13) * the whole document * | 1-9 | INV. A61M1/00 |
| A | US 5 665 080 A (VANDENBERG JAMES T [US]) 9 September 1997 (1997-09-09) * the whole document * | 1-9 | |
| A | US 4 455 140 A (JOSLIN JOEL A [US]) 19 June 1984 (1984-06-19) * column 3, line 24 - line 34; figure 1 * | 1-9 | |
| X | US 5 114 415 A (SHEDLOCK SUSAN [US]) 19 May 1992 (1992-05-19) * the whole document * | 1-9 | |
| X | US 7 955 318 B1 (SCHULTZ JOSEPH P [US] ET AL) 7 June 2011 (2011-06-07) * the whole document * | 1-9 | |
| | | | TECHNICAL FIELDS SEARCHED (IPC) A61M |

The present search report has been drawn up for all claims

| Place of search | Date of completion of the search | Examiner |
|---|---|---|
| The Hague | 20 June 2025 | Chevtchik, Natalia |

## ANNEX TO THE EUROPEAN SEARCH REPORT
## ON EUROPEAN PATENT APPLICATION NO.

EP 25 16 6428

This annex lists the patent family members relating to the patent documents cited in  the above-mentioned European search report.
The members are as contained in the European Patent Office EDP file on
The European Patent Office is in no way liable for these particulars which are merely given for the purpose of information.

20-06-2025

| Patent document cited in search report | | Publication date | Patent family member(s) | Publication date |
|---|---|---|---|---|
| US 5921970 | A | 13-07-1999 | NONE | |
| US 5665080 | A | 09-09-1997 | NONE | |
| US 4455140 | A | 19-06-1984 | NONE | |
| US 5114415 | A | 19-05-1992 | NONE | |
| US 7955318 | B1 | 07-06-2011 | NONE | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62642693